# EUROPÄISCHE PATENTSCHRIFT

(11) **EP 2 381 768 B1**
(45) Veröffentlichungstag und Bekanntmachung des Hinweises auf die Patenterteilung: **04.05.2016**
(21) Anmeldenummer: 09774906.3
(22) Anmeldetag: 15.12.2009
(51) Int. Cl.: A01N 25/02, A01N 25/04, C07C 69/28, A01N 43/653, A01N 47/24, A01P 3/00

(54) **AGROCHEMISCHE ZUSAMMENSETZUNGEN UMFASSEND VERZWEIGTE ALKOXYALKANOATE**
AGROCHEMICAL COMPOSITIONS COMPRISING BRANCHED ALCOOXYALKANOATES
COMPOSITIONS AGROCHIMIQUES COMPRENANT DES ALCOXYALCANOATES RAMIFIÉS

(30) Priorität: 29.12.2008 EP 08173004
(43) Veröffentlichungstag der Anmeldung: 02.11.2011
(73) Patentinhaber: BASF SE, 67056 Ludwigshafen am Rhein (DE)
(72) Erfinder: SOWA, Christian, 67435 Neustadt (DE)
(74) Vertreter: BASF IP Association
(86) Internationale Anmeldenummer: PCT/EP2009/067137
(87) Internationale Veröffentlichungsnummer: WO 2010/076183

(56) Entgegenhaltungen:
- EP-A- 1 151 667
- EP-A- 1 911 349
- WO-A-02/45507
- WO-A-2007/110435
- DATABASE WPI Section Ch, Week 200429 Thomson Scientific, London, GB; Class C03, AN 2004-310503 XP002530050, & JP 2004 107214 A (HOKKO CHEM IND CO) 8. April 2004 (2004-04-08)
- GRIGORIADOU ET AL: "Molecular indicators for pollution source identification in marine and terrestrial water of the industrial area of Kavala city, North Greece", ENVIRONMENTAL POLLUTION, BARKING, GB, Bd. 151, Nr. 1, 3. Dezember 2007 (2007-12-03), Seiten 231-242, XP022373733, ISSN: 0269-7491 in der Anmeldung erwähnt

## Beschreibung

Gegenstand der vorliegenden Erfindung ist eine flüssige agrochemische Zusammensetzung umfassend Pestizid, Formulierungshilfsmittel und Alkoxyalkanoat, sowie ein Verfahren zur Herstellung dieser Zusammensetzung. Die Erfindung betrifft ferner ein Alkoxyalkanoat der Formel V

R³-C(O)-(O-R⁴)ₙ-OH V

wobei R³, R⁴ und n die in den Ansprüchen angegebenen Bedeutungen haben.
Ferner betrifft die Erfindung die Verwendung der Alkoxyalkanoate V in agrochemischen Formulierungen. Weiterer Gegenstand ist die Verwendung der erfindungsgemäßen Zusammensetzung zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen. Des weiteren ist Gegenstand die Verwendung einer erfindungsgemäßen Zusammensetzung zur Bekämpfung von unerwünschtem Insekten- oder Milbenbefall auf Pflanzen und/oder zur Bekämpfung von phytopathogenen Pilzen und/oder zur Bekämpfung unerwünschten Pflanzenwuchs, wobei man Saatgüter von Nutzpflanzen mit der Zusammensetzung behandelt. Schließlich betrifft die vorliegende Erfindung auch Saatgut, behandelt mit der erfindungsgemäßen Zusammensetzung. Kombinationen bevorzugter Merkmale mit anderen bevorzugten Merkmalen werden von der vorliegenden Erfindung umfasst.

Agrochemische Zusammensetzungen umfassend Pestizid, Formulierungshilfsmittel und Alkoxyalkanoat sind allgemein bekannt:
WO 2004/010782 A2 offenbart ein Verfahren zum Bekämpfen von Viren in Pflanzen umfassend das in Kontakt bringen der Pflanzen mit einer Zusammensetzung enthaltend einen Carbonsäureester mit mindestens 14 Kohlenstoffatomen. Als Estergruppen werden beispielsweise Ethylenglykol, Propylenglykol, Polyethylenglykol und Polypropylenglykol aufgelistet. Die Zusammensetzung enthält weiterhin ein nichtionisches Tensid und optional ein Pestizid.
JP 2005 112 727 A offenbart ein Mittel zur Behandlung von Pfirsichblüten enthaltend einen Fettsäureester einer C₈ bis C₁₆ Fettsäure. Der Alkoholteil des Esters kann beispielsweise Ethylenglykol oder Propylenglykol sein. Die Fettsäure kann beispielsweise eine 2-Ethylhexylgruppe sein.
JP 09 059 210 A offenbart ein Lösungsmittel umfassend 99-51 % eines Esters einer C2-11 aliphatischen Säure mit verzweigtem Propylenglykol. Das Lösungsmittel ist für agrochemische Zusammensetzungen geeignet.
WO 2007/110435 offenbart eine wässrige Mikroemulsion umfassend ein Pyrethroid, ein organisches Lösungsmittel und eine Tensidmischung. Als Lösungsmittel werden unter anderem C₁-C₄ Alkylester von Polyalkylenoxiden beschrieben, die 1, 2 oder 3 Alkylenoxidgruppen haben.
EP 1 911 349 A2 offenbart eine Methode zur Erhöhung des Ertrags von landwirtschafltlichen Produkten, indem diese behandelt werden mit einem Alkoxy-(C₁₂₋₃₀)alkanoat.
JP 2004107214 A offenbart eine auf dem Wasser schwimmende agrochemische Formulierung, umfassend ein Tensid, ein hochsiedendes Lösungsmittel, ein hohler, auf Wasser schwimmender Körper und Glaspulver. Als Tensid werden Ethylenglykolmonoester von Oktan-, Nonan-, oder Dekansäure offenbart.

Alkoxyalkanoate sind ebenfalls allgemein bekannt:
Grigoriadou et al. offenbart in Environmetal Pollution 2008, 151, 231-242 2-Ethylhexansäure-2-hydroxypropylester (Formel 9), welcher bei der Analyse von Wasserproben identifiziert wurde. Eine Synthese oder Isolierungsmethode wird nicht offenbart.
EP 0 862 861 A1 offenbart eine insektizide Zusammensetzung umfassend einen Fettsäureester ausgewählt beispielsweise aus Propylenglykolmonolaurat.
WO 99/30559 A1 offenbart eine Methode zum Abtöten von Neamtodeneiern durch Anwendung einer Zusammensetzung enthaltend eine C₈ bis C₁₄ Fettsäureester. Die Estergruppe ist bevorzugt Ethylenglykol.
EP 1 151 667 A2 offenbart eine Pflanzen aktivierende Verbindung ausgewählt beispielsweise aus Estern von linearen oder verzweigten C₁₂ bis C₃₀ Säuren mit Polyethylenglykol oder Polypropylenglykol.
WO 2001/34898 offenbart wässrige Lösungen enthaltend ein aromatisches Formaldehyd-Kondensationsprodukt, eine aliphatische Carbonsäure, eine aromatische Sulfonsäure und ein Lösungsmittel, welches beispielsweise ein C₁-C₆ Alkylester von Polyalkylenoxiden mit 1 bis 10 Alkylenoxideinheiten sein kann.

Aufgabe der vorliegenden Erfindung war eine Zusammensetzung umfassend Pestizid bereitzustellen, welche eine hohe Beladung mit Pestizid ermöglicht und dabei stabil ist. Stabil bedeutet dabei, dass die Zusammensetzung und insbesondere die mit Wasser verdünnte Zusammensetzung nicht oder wenig zur Kristallisation neigt. Weiterhin war es Aufgabe, dass die vorgenannte Zusammensetzung sowohl für gelöste oder suspendierte Pestizide die geringe Kristallisationsneigung aufweist. Des weiteren war es Aufgabe, eine Zusammensetzung umfassend Pestizid in Form eines Emulsionskonzentrats bereitzustellen, das wenig zur Kristallisationsneigung neigt.

Die Aufgabe wurde gelöst durch eine flüssige, agrochemische Zusammensetzung umfassend Pestizid, Formulierungshilfsmittel und Alkoxyalkanoat, wobei das Alkoxyalkanoat der Formel I

R¹-C(O)-(O-R²)ₙ-OH I

wobei R¹ für ein verzweigtes C₇-C₁₁-Alkyl,
R² für ein C₂-C₄-Alkylen, und
n für 1 bis 3 steht,
entspricht, wobei das Pestizid bei 20 °C zu mindestens 10 g/l löslich im Alkoxyalkanoat ist.
R¹ steht für eine verzweigte C₇-C₁₁-Alkylgruppe. Das bedeutet, dass der Alkylrest 7 bis 11 Kohlenstoffatome hat und dabei mindestens eine, bevorzugt eine bis drei, Verzweigung in der Kohlenstoffkette aufweist, wobei das Pestizid bei 20 °C zu mindestens 10 g/l löslich im Alkoxyalkanoat ist. R¹ kann gesättigt oder ungesättigt sein, bevorzugt ist es gesättigt. R¹ kann auch cyclische Strukturen beinhalten, bevorzugt enthält R¹ keine cyclischen Strukturen. R¹ steht bevorzugt für ein verzweigtes C₈ bis C₉ Alkyl.

In einer bevorzugten Ausführungsform steht R¹ für eine Struktur der Formel A bis J, 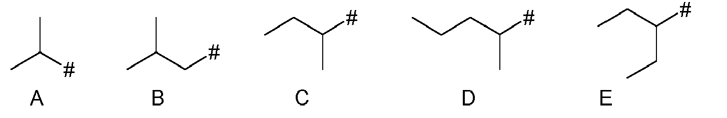 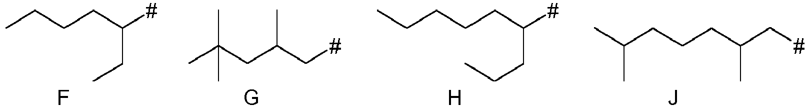 wobei # die Bindung zur Carbonylgruppe von Formel I kennzeichnet. R¹ ist insbesondere eine Struktur der Formel F, G oder H.

R² steht für C₂-C₄-Alkylen, beispielsweise -CH₂-CH₂-, -CH₂-CH(CH₃)-, -CH(CH₃)-CH₂-, -CH₂-CH(CH₂CH₃)-, -CH₂-CH₂-CH₂-, oder -CH₂-CH₂-CH₂-CH₂-. R² steht bevorzugt für C₂-C₃-Alkylen, beispielsweise -CH₂-CH₂- oder -CH₂-CH(CH₃)-.

Der Index n kann in einem bestimmten Wertebereich liegen, beispielsweise 1 bis 3. Das bedeutet, dass sowohl ganzzahlige Werte, wie 1, 2 oder 3, als auch Werte dazwischen, wie 1,15, vorkommen können. Bevorzugt steht n für 1 bis 2, besonders bevorzugt für 1. In einer weiteren bevorzugten Ausführungsform wird das Alkoxyalkanoat durch Alkoxylierung einer Säure hergestellt und n steht für 1,1 bis 2,9, bevorzugt für 1,3 bis 2,6.

Geeignete Alkoxyalkanoate I sind beispielsweise die Verbindungen der Formeln II, III und IV, 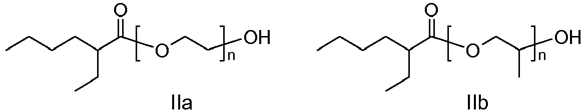 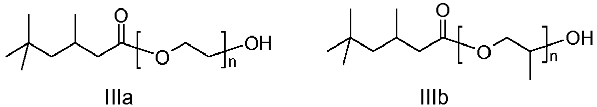 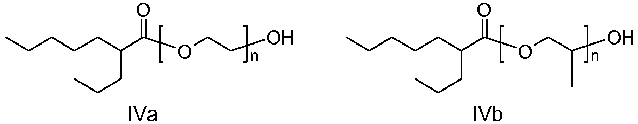 wobei n für einen Wert von 1 bis 3, bevorzugt von 1 bis 2 und insbesondere 1 steht.

Die erfindungsgemäße Zusammensetzung enthält üblicherweise mindestens 20 Gew.%, bevorzugt mindestens 30 Gew.%, besonders bevorzugt mindestens 40 Gew.% Alkoxyalkanoat der Formel I bezogen auf die Zusammensetzung. Meist enthält die Zusammensetzung höchstens 95 Gew.%, bevorzugt höchstens 90 Gew.%, besonders bevorzugt höchstens 80 Gew.% Alkoxyalkanoat I.

Der Begriff Pestizid bezeichnet mindestens einen Wirkstoff ausgewählt aus der Gruppe der Fungizide, Insektizide, Nematizide, Herbizide, Safener und/oder Wachstumsregulatoren. Bevorzugte Pestizide sind Fungizide, Insektizide und Herbizide, insbesondere Fungizide. Auch Mischungen von Pestiziden aus zwei oder mehr der vorgenannten Klassen können verwendet werden. Der Fachmann ist vertraut mit solchen Pestiziden, die beispielsweise in Pesticide Manual, 14th Ed. (2006), The British Crop Protection Council, London, gefunden werden können. Geeignete Insektizide sind Insektizide der Klasse der Carbamate, Organophophate, Organochlor-Insektizide, Phenylpyrazole, Pyrethroide, Neonicotinoide, Spinosine, Avermectine, Milbemycine, Juvenil Hormon Analoga, Alkylhalide, Organozinn-Verbindungen, Nereistoxin-Analoga, Benzoylharnstoffe, Diacylhydrazine, METI Akarizide, sowie Insektizide wie Chloropicrin, Pymetrozin, Flonicamid, Clofentezin, Hexythiazox, Etoxazol, Diafenthiuron, Propargit, Tetradifon, Chlorfenapyr, DNOC, Buprofezin, Cyromazin, Amitraz, Hydramethylnon, Acequinocyl, Fluacrypyrim, Rotenon, oder deren Derivate. Geeignete Fungizide sind Fungizide der Klassen Dinitroaniline, Allylamine, Anilinopyrimidine, Antibiotica, aromatische Kohlenwasserstoffe, Benzenesulfonamide, Benzimidazole, Benzisothiazole, Benzophenone, Benzothiadiazole, Benzotriazine, Benzylcarbamate, Carbamates, Carboxamide, Carbonsäureamdide, Chloronitrile, Cyanoacetamideoxime, Cyanoimidazole, Cyclopropanecarboxamide, Dicarboximide, Dihydrodioxazine, Dinitrophenylcrotonate, Dithiocarbamate, Dithiolane, Ethylphosphonate, Ethylaminothiazolcarboxamide, Guanidines, Hydroxy-(2-amino-)pyrimidine, Hydroxyanilides, Imidazole, Imidazolinone, A-norganika, Isobenzofuranone, Methoxyacrylate, Methoxycarbamates, Morpholines, N-Phenylcarbamate, Oxazolidinedione, Oximinoacetate, Oximinoacetamide, Peptidylpyrimidinnucleoside, Phenylacetamide, Phenylamide, Phenylpyrrole, Phenylharnstoffe, Phosphonate, Phosphorothiolate, Phthalamsäuren, Phthalimide, Piperazine, Piperidine, Propionamide, Pyridazinone, Pyridine, Pyridinylmethylbenzamide, Pyrimidinamine, Pyrimidine, Pyrimidinonehydrazone, Pyrroloquinolinone, Quinazolinone, Chinoline, Chinone, Sulfamide, Sulfamoyltriazole, Thiazolecarboxamide, Thiocarbamate, Thiocarbamate, Thiophanate, Thiophenecarboxamide, Toluamide, Triphenylzinn Verbindungen, Triazine, Triazole. Geeignete Herbizide sind Herbizide der Klassen der Acetamide, Amide, Aryloxyphenoxypropionate, Benzamide, Benzofuran, Benzoesäuren, Benzothiadiazinone, Bipyridylium, Carbamate, Chloroacetamide, Chlorcarbonsäuren, Cyclohexanedione, Dinitroaniline, Dinitrophenol, Diphenylether, Glycine, Imidazolinone, Isoxazole, Isoxazolidinone, Nitrile, N-phenylphthalimide, Oxadiazole, Oxazolidinedione, Oxyacetamide, Phenoxycarbonsäuren, Phenylcarbamate, Phenylpyrazole, Phenylpyrazoline, Phenylpyridazine, Phosphinsäuren, Phosphoroamidate, Phosphorodithioate, Phthalamate, Pyrazole, Pyridazinone, Pyridine, Pyridincarbonsäuren, Pyridinecarboxamide, Pyrimidindione, Pyrimidinyl(thio)benzoate, Chinolincarbonsäuren, Semicarbazone, Sulfonylaminocarbonyltriazolinone, Sulfonylharnstoffe, Tetrazolinone, Thiadiazole, Thiocarbamate, Triazine, Triazinone, Triazole, Triazolinone, Triazolinone, Triazolocarboxamide, Triazolopyrimidine, Triketone, Uracile, Harnstoffe.

In einer Ausführungsform enthält das Pestizid ein Insektizid, bevorzugt besteht das Pestizid aus mindestens einem Insektizid. In einer weiteren Ausführungsform enthält das Pestizid ein Fungizid, bevorzugt besteht das Pestizid aus mindestens einem Fungizid. Bevorzugte Fungizide sind Pyraclostrobin, Metconazol und Epoxiconazol. In einer weiteren Ausführungsform enthält das Pestizid ein Herbizid, bevorzugt besteht das Pestizid aus mindestens einem Herbizid. In einer weiteren Ausführungsform enthält das Pestizid ein Wachstumsregulator, bevorzugt besteht das Pestizid aus mindestens einem Wachstumsregulator.

Das Pestizid ist bei 20 °C bevorzugt zu mindestens 30 g/l und besonders bevorzugt zu mindestens 50 g/l löslich im Alkoxyalkanoat. Als Lösungsmittelsystem wird dabei das jeweils verwendete Alkoxyalkanoat eingesetzt.

In einer weiteren Ausführungsform liegt das Pestizid in gelöster Form in der flüssigen, agrochemischen Zusammensetzung vor. Bevorzugt ist das Pestizid bei 20 ° zu mindestens 90 Gew.%, bevorzugt zu mindestens 98 Gew.%, bezogen auf das Pestizid, im Lösungsmittelsystem gelöst.

In einer weiteren Ausführungsform ist mindestens ein Pestizid zu mindestens 90 Gew.%, bezogen auf das Pestizid, im Lösungsmittelsystem in Form fester Teilchen suspendiert. Falls die Zusammensetzung mindestens zwei Pestizide umfasst, ist mindestens ein Pestizid zu mindestens 90 Gew.% im Lösungsmittelsystem gelöst. Bevorzugt ist das Pestizid zu mindestens 95 Gew.%, besonders bevorzugt zu mindestens 98 Gew.% im Lösungsmittelsystem suspendiert.

Die erfindungsgemäße Zusammensetzung umfasst üblicherweise 0.1 bis 70 Gew.% Pestizid, bevorzugt 1 bis 50 Gew. %, insbesondere 3 bis 30 Gew.%, bezogen auf die Zusammensetzung.

Die erfindungsgemäße Zusammensetzung enthält Formulierungshilfsmittel, wobei sich die Wahl der Hilfsmittel üblicherweise nach der konkreten Anwendungsform bzw. dem Wirkstoff richtet. Beispiele für geeignete Formulierungshilfsmittel sind zusätzliche Lösungsmittel, Tenside und andere oberflächenaktive Stoffe (wie Solubilisatoren, Schutzkolloide, Netzmittel und Haftmittel), Adjuvantien, organische und anorganische Verdicker, Bakterizide, Frostschutzmittel, Entschäumer, Farbstoffe und Kleber (z. B. für Saatgutbehandlung).

Als zusätzliche Lösungsmittel, die in der Zusammensetzung zusätzlich zum Lösungsmittel Alkoxyalkanoat der Formel I enthalten sein können, kommen organische Lösungsmittel wie Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol, Benzylalkohol und Cyclohexanol, Glykole, Ketone wie Cyclohexanon, gamma-Butyrolacton, Dimethylfettsäureamide, Fettsäuren und Fettsäureester und stark polare Lösungsmittel, z.B. Amine wie N-Methylpyrrolidon, in Betracht. Bevorzugt werden Alkohole, wie Benzylalkohol. Grundsätzlich können auch Lösungsmittelgemische verwendet werden. Bevorzugt werden der erfindungsgemäßen Zusammensetzung höchstens 40 Gew.%, bevorzugt höchstens 20 Gew.%, jeweils bezogen auf die Zusammensetzung, zugesetzt.

Tenside können einzeln oder in Mischung eingesetzt werden. Tenside sind Verbindungen, welche die Oberflächenspannung von Wasser verringern. Beispiele für Tenside sind ionische (anionisch oder kationisch) und nicht-ionische Tenside. Bevorzugt umfasst die Zusammensetzung mindestens zwei Tenside, besonders bevorzugt umfasst sie ein nichtionisches Tensid und ein anionisches Tensid. Das Mengenverhältnis von nichtionischem zu anionischem Tensid beträgt meist 1:5 bis 5:1, bevorzugt 1:3 bis 3:1.

Geeignete ionische Tenside sind die Alkali-, Erdalkali-, Ammoniumsalze von aromatischen Sulfonsäuren, z. B. von Lignin-(Borresperse^{®}-Typen, Borregaard, Norwegen), Phenol-, Naphthalin-(Morwet^{®}-Typen, Akzo Nobel, USA) und Dibutylnaphthalinsulfonsäure (Nekal^{®}-Typen, BASF, Deutschland), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polycarboxylate (Sokalan^{®}-Typen, BASF, Deutschland) oder Phosphatester alkoxylierter Alkohole.

Bevorzugte ionische Tenside sind anionische Tenside. Geeignete anionische Tenside sind Alkalimetall- und Ammoniumsalze von Alkylsulfaten (Alkylrest: C8 bis C12), von Schwefelsäurehalbestern ethoxylierter Alkanole (Ethoxilierungsgrad von 4 bis 30, Alkylrest: C12 bis C18) und ethoxylierter Alkylphenole (Ethoxilierungsgrad von 3 bis 50, Alkylrest: C4 bis C12), von Alkylsulfonsäuren (Alkyl-rest: C12 bis C18) und von Alkylarylsulfonsäuren (Alkylrest: C9 bis C18), oder Phosphatester eines alkoxylierten Alkohols, speziell Phosphatester eines ethoxyliertem C10-16 Fettalkohols mit Ethoxilierungsgrad von 3 bis 15. Als weitere anionische Temsode sind Verbindungen der allgemeinen Formel (I) geeignet, 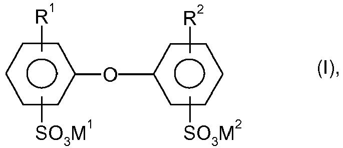 worin R1 und R2 H-Atome oder C4- bis C24-Alkyl bedeuten und nicht gleichzeitig H-Atome sind, und M1 und M2 Alkalimetallionen und/oder Ammoniumionen sein können, erwiesen. In der allgemeinen Formel (I) bedeuten R1 und R2 bevorzugt lineare oder verzweigte Alkylreste mit 6 bis 18 C-Atomen, insbesondere mit 6, 12 und 16 C-Atomen oder Wasserstoff, wobei R1 und R2 nicht beide gleichzeitig H-Atome sind. M1 und M2 sind bevorzugt Natrium, Kalium oder Ammonium, wobei Natrium besonders bevorzugt ist. Besonders vorteilhaft sind Verbindungen (I), in denen M1 und M2 Natrium, R1 ein verzweigter Alkylrest mit 12 C-Atomen und R2 ein H-Atom oder R1 ist. Häufig werden technische Gemische verwendet, die einen Anteil von 50 bis 90 Gew.-% des monoalkylierten Produktes aufweisen, wie beispielsweise Dowfax® 2A1 (Marke der Dow Chemical Company). Bevorzugte anionische Tenside sind Alkalimetall- und Ammoniumsalze von Alkylarylsulfonsäuren (Alkylrest: C9 bis C18), bevorzugt lineare oder verzweigte Alkylbenzolsulfonsäuren, und Phosphatester eines ethoxyliertem C10-16 Fettalkohols mit Ethoxilierungsgrad von 3 bis 15.

Geeignete nicht-ionische Tenside sind Polyoxyethylenoctylphenolether, alkoxilierte Alkohole wie ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol^{®}-Typen, Clariant), , Polyalkoxylate, Polyvinylamin (Lupamin^{®}-Typen, BASF SE), Polyethylenimin (Lupasol^{®}-Typen, BASF SE), Polyvinylpyrrolidon und deren Copolymere oder Blockpolymere. Geeigneter alkoxylierter Alkohol ist bevorzugt ein mit Ethylenoxid (EO) oder Propylenoxid (PO) alkoxylierter Fettalkohol, insbesondere mit 8 bis 32, speziell mit 9 bis 18 Kohlenstoffatomen im Fettalkoholrest. Der alkoxylierte Fettalkohol hat meist einen Ethoxylierungsgrad von 1 bis 30, bevorzugt 2 bis 10 und speziell 4 bis 8 Ethylenoxidgruppen und/oder ein Propoxilierungsgrad von 1 bis 30, bevorzugt 2 bis 15, und speziell 3 bis 10 Propylenoxidgruppen. Das Blockpolymer ist üblicherweise ein Di- oder Triblockpolymer oder dessen Derivat, wobei der polymere Anteil aufgebaut aus Ethylenoxid und Propylenoxid. Die mittlere Molmasse liegt meist bei mindestens 1000 g/mol, bevorzugt mindestens 2000 g/mol. Speziell geeignet ist Poly(ethylenoxid-block-propylenoxid)-alkylether mit einer Molmasse von mindestens 2000 g/mol und einem C₁₋₁₀-Alkylethereinheit. Bevorzugte nichtionische Tenside sind Alkylphenolpolyglykolether, Tristyrylphenolethoxylate, ethoxyliertes Rizinusöl, bevorzugt mit jeweils 10 bis 40 Ethylenoxideinheiten pro Molekül.

Als oberflächenaktive Stoffe (Adjuvantien, Netz-, Haft-, Dispergier- oder Emulgiermittel) zusätzlich zu den vorgenannten Tensiden kommen die Alkali-, Erdalkali-, Ammoniumsalze von aromatischen Sulfonsäuren, z. B. von Lignin-(Borresperse^{®}-Typen, Borregaard, Norwegen), Phenol-, Naphthalin-(Morwet^{®}-Typen, Akzo Nobel) und Dibutylnaphthalinsulfonsäure (Nekal^{®}-Typen, BASF), sowie von Fettsäuren, Alkyl- und Alkylarylsulfonaten, Alkyl-, Laurylether- und Fettalkoholsulfaten, sowie Salze sulfatierter Hexa-, Hepta- und Octadecanole sowie von Fettalkoholglykolethern, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen sowie Proteine, denaturierte Proteine, Polysaccharide (z.B. Methylcellulose), hydrophob modifizierte Stärken, Polyvinylalkohol (Mowiol^{®}-Typen, Clariant), Polycarboxylate (Sokalan^{®}-Typen, BASF), Polyalkoxylate, Polyvinylamin (Lupamin^{®}-Typen, BASF), Polyethylenimin (Lupasol^{®}-Typen, BASF), Polyvinylpyrrolidon und deren Copolymere in Betracht.

Die erfindungsgemäße Zusammensetzung kann hohe Mengen oberflächenaktiver Stoffe und Tensid umfassen. Sie kann 0,1 bis 40 Gew.%, bevorzugt 1 bis 30 und insbesondere 2 bis 20 Gew.% Gesamtmenge von oberflächenaktiven Stoffe und Tensiden umfassen bezogen auf die Gesamtmenge der Zusammensetzung.

Beispiele für Adjuvantien sind organisch modifizierte Polysiloxane, wie BreakThruS 240^{®}; Alkoholalkoxylate, wie Atplus^{®}245, Atplus^{®}MBA 1303, Plurafac^{®}LF und Lutensol^{®} ON ; EO-PO-Blockpolymerisate, z. B. Pluronic^{®} RPE 2035 und Genapol^{®} B; Alkoholethoxylate, z. B. Lutensol^{®} XP 80; und Natriumdioctylsulfosuccinat, z. B. Leophen^{®} RA. Beispiele für Verdicker (d. h. Verbindungen, die der Zusammensetzung ein modifiziertes Fließverhalten verleihen, d. h. hohe Viskosität im Ruhezustand und niedrige Viskosität im bewegten Zustand) sind Polysaccharide sowie organische und anorganische Schichtmineralien wie Xanthan Gum (Kelzan^{®}, CP Kelco), Rhodopol^{®} 23 (Rhodia) oder Veegum^{®} (R.T. Vanderbilt) oder Attaclay^{®} (Engelhard Corp.).

Bakterizide können zur Stabilisierung der Zusammensetzung zugesetzt werden. Beispiele für Bakterizide sind solche basierend auf Diclorophen und Benzylalkoholhemiformal (Proxel^{®} der Fa. ICI oder Acticide^{®} RS der Fa. Thor Chemie und Kathon^{®} MK der Fa. Rohm & Haas) sowie Isothiazolinonderivaten wie Alkylisothiazolinonen und Benzisothiazolinonen (Acticide^{®} MBS der Fa. Thor Chemie).

Beispiele für geeignete Frostschutzmittel sind Ethylenglycol, Propylenglycol, Harnstoff und Glycerin.

Beispiele für Entschäumer sind Silikonemulsionen (wie z. B. Silikon^{®} SRE, Wacker, Deutschland oder Rhodorsil^{®}, Rhodia, Frankreich), langkettige Alkohole, Fettsäuren, Salze von Fettsäuren, fluororganische Verbindungen und deren Gemische.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C. I. Pigment Red 112 und C. I. Solvent Red 1, Pigment blue 15:4, Pigment blue 15:3, Pigment blue 15:2, Pigment blue 15:1, Pigment blue 80, Pigment yellow 1, Pigment yellow 13, Pigment red 48:2, Pigment red 48:1, Pigment red 57:1, Pigment red 53:1, Pigment orange 43, Pigment orange 34, Pigment orange 5, Pigment green 36, Pigment green 7, Pigment white 6, Pigment brown 25, Basic violet 10, Basic violet 49, Acid red 51, Acid red 52, Acid red 14, Acid blue 9, Acid yellow 23, Basic red 10, Basic red 108 bekannten Farbstoffe und Pigmente.

Beispiele für Kleber sind Polyvinylpyrrolidon, Polyvinylacetat, Polyvinylalkohol und Celluloseether (Tylose^{®}, Shin-Etsu, Japan).

Üblicherweise liegen erfindungsgemäßen Zusammensetzungen in Form agrochemischer Formulierungen vor. Geeignete agrochemische Formulierungen sind wasserlösliche Konzentrate (SL, LS), redispergierbare Konzentrate (DC), emulgierbare Konzentrate (EC), Emulsionen (EW, EO, ES, ME), Suspensionen (SC, OD, FS) oder Suspoemulsionen (SE) vor. Bevorzugt liegt die Zusammensetzung in Form eines emulgierbaren Konzentrates (EC) vor. In einer bevorzugten Ausführungsform liegt die erfindungsgemäße Zusammensetzung in Form einer flüssigen Zusammensetzung vor, wie wasserlösliche Konzentrate (SL, LS), redispergierbare Konzentrate (DC), emulgierbare Konzentrate (EC), Emulsionen (EW, EO, ES, ME), Suspensionen (SC, OD, FS) oder Suspoemulsionen (SE).

Die erfindungsgemäße Zusammensetzung wird meist vor der Anwendung verdünnt um den sog. Tankmix herzustellen. Zur Verdünnung kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Isophoron, stark polare Lösungsmittel, z.B. Dimethylsulfoxid, N-Methylpyrrolidon oder Wasser in Betracht. Bevorzugt wird Wasser verwendet. Die verdünnte Zusammensetzung wird üblicherweise durch Versprühen oder Vernebeln angewendet. Zu dem Tankmix können Öle verschiedenen Typs, Netzmittel, Adjuvants, Herbizide, Bakterizide, Fungizide unmittelbar vor der Anwendung (Tankmix) zugesetzt werden. Diese Mittel können zu den erfindungsgemäßen Zusammensetzungen im Gewichtsverhältnis 1:100 bis 100:1, bevorzugt 1:10 bis 10:1 zugemischt werden. Die Pestizidkonzentration im Tankmix kann in größeren Bereichen variiert werden. Im Allgemeinen liegen sie zwischen 0,0001 und 10%, vorzugsweise zwischen 0,01 und 1%. Die Aufwandmengen liegen bei der Anwendung im Pflanzenschutz je nach Art des gewünschten Effektes zwischen 0,01 und 2,0 kg Wirkstoff pro ha.

Die vorliegende Erfindung betrifft auch die Verwendung einer erfindungsgemäßen Zusammensetzung zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt. Weiterhin betrifft die Erfindung die Verwendung einer erfindungsgemäßen Zusammensetzung zur Bekämpfung von unerwünschtem Insekten- oder Milbenbefall auf Pflanzen und/oder zur Bekämpfung von phytopathogenen Pilzen und/oder zur Bekämpfung unerwünschten Pflanzenwuchs, wobei man Saatgüter von Nutzpflanzen mit der Zusammensetzung behandelt.

Des weiteren betrifft die Erfindung Saatgut, das mit einer erfindungsgemäßen Zusammensetzung behandelt wurde. Bevorzugt wurde das Saatgut mit der erfindungsgemäßen Zusammensetzung gebeizt. Beizen bedeutet, dass das Saatgut mit der Zusammensetzung behandelt wurde und die Zusammensetzung auf dem Saatgut verbleibt. Diese Zusammensetzung kann auf das Saatgut unverdünnt oder, bevorzugt, verdünnt angewendet werden. Hierbei kann die entsprechende Zusammensetzung 2 bis 10-fach verdünnt werden, so dass in den für die Beize zu verwendeten Zusammensetzungen 0,01 bis 60% Gew.-%, vorzugsweise 0,1 bis 40% Gew.-% Pestizid vorhanden sind. Die Anwendung kann vor der Aussaat erfolgen. Die Behandlung von pflanzlichem Vermehrungsmaterial, insbesondere die Behandlung von Saatgut, sind dem Fachmann bekannt, und erfolgen durch Bestäuben, Beschichten, Pelletieren, Eintauchen oder Tränken des pflanzlichen Vermehrungsmaterial, wobei die Behandlung bevorzugt durch Pelletieren, Beschichten und Bestäuben erfolgt, so dass z. B. eine vorzeitige Keimung des Saatguts verhindert wird. Bei der Behandlung von Saatgut werden im allgemeinen Pestizidmengen von 1 bis 1000 g/100 kg, vorzugsweise 5 bis 100 g/100 kg Vermehrungsmaterial bzw. Saatgut verwendet.

Die vorliegende Erfindung betrifft auch ein Verfahren zur Herstellung einer erfindungsgemäßen Zusammensetzung, wobei man ein Pestizid und ein Alkoxyalkanoat I mischt. Bevorzugte Alkoxyalkanoat I und Lösungsmittel sind wie oben beschrieben. Das Vermischen erfolgt durch übliche Mischverfahren, wie Rühren, Schütteln, oder sonstigen Energieeintrag. Weitere Hilfsmittel, die zur Herstellung von agrochemischen Formulierungen eingesetzt werden, können in üblichen Mengen zugesetzt werden. Beispiele geeigneter Formulierungshilfsmittel sind wie oben beschrieben.

Die vorliegende Erfindung betrifft ferner Alkoxyalkanoate der Formel V

R³-C(O)-(O-R⁴)ₙ-OH V

wobei R⁴ für ein C₂-C₄-Alkylen, und
n für 1 bis 3 steht.

R³ steht für eine Struktur der Formel G bis J, 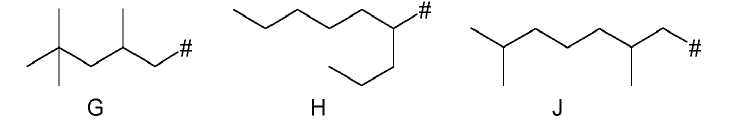 wobei # die Bindung zur Carbonylgruppe von Formel I kennzeichnet. R³ ist insbesondere eine Struktur der Formel G oder H.

R⁴ steht für C₂-C₄-Alkylen, beispielsweise -CH₂-CH₂-, -CH₂-CH(CH₃)-, -CH(CH₃)-CH₂-, -CH₂-CH(CH₂CH₃)-, -CH₂-CH₂-CH₂-, oder -CH₂-CH₂-CH₂-CH₂-. R⁴ steht bevorzugt für C₂-C₃-Alkylen, beispielsweise -CH₂-CH₂- oder -CH₂-CH(CH₃)-.

Der Index n kann in einem bestimmten Wertebereich liegen, beispielsweise 1 bis 3. Das bedeutet, dass sowohl ganzzahlige Werte, wie 1, 2 oder 3, als auch Werte dazwischen, wie 2,15, vorkommen können. Bevorzugt steht n für 1 bis 2, besonders bevorzugt für 1. In einer weiteren bevorzugten Ausführungsform wird das Alkoxyalkanoat durch Alkoxylierung einer Säure hergestellt und n steht für 1,1 bis 2,9, bevorzugt für 1,3 bis 2,6.

Besonders bevorzugte Alkoxyalkanoate V sind beispielsweise die Verbindungen der Formeln II, III und IV.

Die Pyrrolidonalkylenoxide der Formel I bis V können durch Alkoxilierung von Pyrrolidon hergestellt werden. Zur Alkoxylierung können Ethylenoxid, Propylenoxid und Butylenoxid verwendet werden. Die Alkoxylierung kann durch starke Basen, wie Alkalihydroxide und Erdalkalihydroxi- de, Brönstedsäuren oder Lewissäuren, wie AlCl₃, BF₃ katalysiert werden. Für eng verteilte Alkoholoxylate können Katalysatoren wie Hydrotalcit oder Doppelmetallcyanide (DMC) verwendet werden. Die Alkoxilierung wird vorzugsweise bei Temperaturen von etwa 90 bis 240 °C, besonders bevorzugt von 110 bis 190 °C durchgeführt. Das Alkylenoxid oder die Mischung verschiedener Alkylenoxide wird Pyrrolidon und Katalysator unter dem bei der gewählten Reaktionstemperatur herrschenden Dampfdruck des Alkylenoxidgemischs oder einem höheren Druck zugeführt. Gewünschtenfalls kann das Alkylenoxid mit einem Inertgas (beispielsweise Edelgase, Stickstoff, CO₂) bis zu 99,9 % verdünnt werden. Dadurch wird insbesondere im Fall des Ethylenoxids eine zusätzliche Sicherheit gegen den Gasphasenzerfall dieses Alkylen- oxids gegeben, wobei bei dieser Ausführungsform auch ein weiteres Alkylenoxid, beispielsweise Propylenoxid, als Inertgas im Sinne der Erfindung verwendet werden kann. Geeignete Alkoxilierungsbedingungen sind auch in Nikolaus Schönfeldt, Grenzflächenaktive Äthylenoxid-Addukte, Wissenschaftliche Verlagsgesellschaft mbH, Stuttgart 1984 beschrieben. In der Regel wird die Alkoxylierung in Gegenwart des Katalysators ohne Zusatz eines Lösungsmittels durchgeführt. Die Alkoxylierung kann jedoch auch unter Mitverwendung eines unter den Alkoxilierungsbedingungen inerten Lösungsmittels durchgeführt werden.

In einer geeigneten Ausführung wird die Alkoxylierung durch wenigstens eine starke Base katalysiert. Geeignete starke Basen sind z. B. Alkalialkoholate, Alkalihydroxide, Erdalkalioxide oder Erdalkalihydroxide. Die Basen werden in der Regel in einer Menge von 0,01 bis 1 Gew.-% bezogen auf die Menge des zu alkoxilierenden Pyrrolidons eingesetzt (vergl. G. Gee et al., J. Chem. Soc.(1961), S. 1345; B. Wojtech, Makromol. Chem. 66, (1966), S. 180). Auch eine saure Katalyse der Alkoxilierungsreaktion ist möglich. Neben Brönstedsäuren eignen sich auch Lewissäuren wie zum Beispiel AlCl₃, BF₃, BF₃-Dietherate, BF₃ x H₃PO₄, SbCl₄ x 2 H₂O, Hydrotalcit (Vgl. P. H. Plesch, The Chemistry of Cationic Polymerization, Pergamon Press, New York (1963)).

Die Alkoxyalkanoate der Formel V sind besonders geeignet als Alkoxyalkanoate in der erfindungsgemäßen Zusammensetzung.

Die vorliegende Erfindung betrifft auch die Verwendung des Alkoxyalkanoate der vorstehend beschriebenen Formel V in agrochemischen Formulierungen. Bevorzugt wird das Alkoxyalkanoat verwendet zum Lösen eines Pestizides in einer agrochemischen Formulierung. Bevorzugte Alkoxyalkanoate sind wie vorstehend beschrieben. Agrochemische Formulierungen sind dem Fachmann bekannt. Sie enthalten üblicherweise ein Pestizid und optional Hilfsmittel für agrochemische Formulierungen, beispielsweise die vorgenannten Hilfsstoffe für agrochemische Formulierungen.

Vorteile der vorliegenden Erfindung sind unter anderem, dass sie eine hohe Beladung einer Zusammensetzung mit Pestizid ermöglicht und dabei stabil ist. Die Zusammensetzung und die mit Wasser verdünnte Zusammensetzung neigen nicht oder wenig zur Kristallisation. Die Zusammensetzung ist sowohl für gelöste als auch suspendierte Pestizide geeignet und weist in beiden Fällen eine geringe Kristallisationsneigung auf. Insbesondere Zusammensetzungen in Form von Emulsionskonzentraten sind stabil und neigen nicht zur Kristallisation.

Nachfolgende Beispiele erläutern die Erfindung ohne sie einzuschränken.

### Beispiele

Tensid 1: Tristyrlyphenolethoxylat mit 16 Mol Ethylenoxid pro Mol (kommerziell erhältlich als Soprophor® BSU von Rhodia S.A.)
Tensid 2: Calciumdodecylbenzolsulfonat (kommerziell erhältlich als Calsogen® AR 100 ND von Clariant).
Tensid 3: Dodecylbenzolsulfosäure, Calciumsalz (kommerziell erhältlich als Wettol® EM 1 von BASF).
Tensid 4: nichtionisches Tensid basierend auf ethoxiliertem Castoröl (kommerziell erhältlich als Wettol® EM 31 von BASF).
Epoxyconazol: Reinheit: 95,5 Gew.%
Metconazol: Reinheit 98,8 Gew.%
Pyraclostrobin: Reinheit 99,4 Gew.%

### Beispiel 1A: 2-Ethylhexansäure + 1 EO

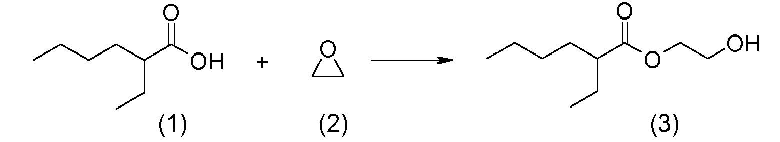

288,4 g (2,0 mol) 2-Ethylhexansäure (1) und 1,5 g Kaliumhydroxid (50 Gew.% in Wasser) wurden bei 90 °C und unter 20 mbar am Rotationsverdampfer unter Stickstoff entwässert. Danach wurde der Ansatz in einen Druckreaktor überführt, der Druck mit Stickstoff auf 1,5 bar eingestellt und 88,0 g (2,0 mol) Ethylenoxid (2) innerhalb 1 h bei 140 °C zudosiert mit einem Druck von maximal 6,2 bar. Nach 15 h Nachrühren ließ man den Ansatz auf 80 °C abkühlen, spülte unter Rühren mit Stickstoff und erhielt 365 g eines hellgelben, klaren Produkts (3). Die H-NMR Analyse bestätigte die Struktur.

### Beispiel 1B: 2-Ethylhexansäure + 1 PO

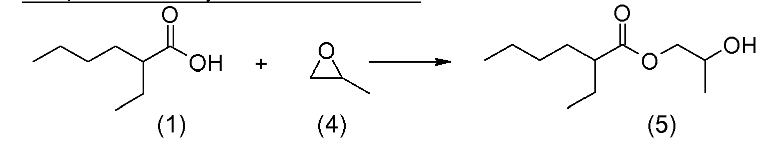

288,4 g (2,0 mol) 2-Ethylhexansäure (1) und 1,6 g Kaliumhydroxid (50 Gew.% in Wasser) wurden bei 90 °C und unter 20 mbar am Rotationsverdampfer unter Stickstoff entwässert. Danach wurde der Ansatz in einen Druckreaktor überführt, der Druck mit Stickstoff auf 1,5 bar eingestellt und 116,2 g (2,0 mol) Propylenoxid (4) innerhalb 1 h bei 140 °C zudosiert mit einem Druck von maximal 4,9 bar. Nach 15 h Nachrühren ließ man den Ansatz auf 80 °C abkühlen, spülte unter Rühren mit Stickstoff und erhielt 399 g eines hellgelben, klaren Produkts (5). Die H-NMR Analyse bestätigte die Struktur.

### Beispiel 1C: Isononansäure + 1 PO

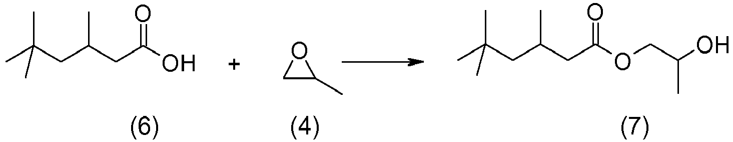

316,5 g (2,0 mol) iso-Nonansäure (6) und 1,74 g Kaliumhydroxid (50 Gew.% in Wasser) wurden bei 90 °C und unter 20 mbar am Rotationsverdampfer unter Stickstoff entwässert. Danach wurde der Ansatz in einen Druckreaktor überführt, der Druck mit Stickstoff auf 1,5 bar eingestellt und 116,2 g (2,0 mol) Propylenoxid (4) innerhalb 1 h bei 140 °C zudosiert mit einem Druck von maximal 4,9 bar. Nach 12 h Nachrühren ließ man den Ansatz auf 80 °C abkühlen, spülte unter Rühren mit Stickstoff und erhielt 440 g eines gelben, klaren Produkts (7). Die H-NMR Analyse bestätigte die Struktur.

### Beispiel 1D: 2-Propylheptansäure + 1 EO

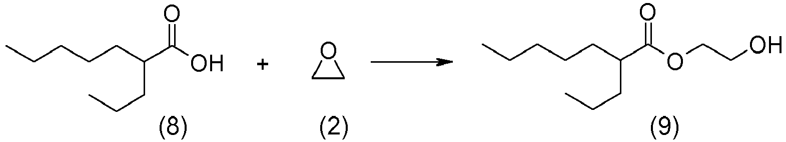

344,6 g (2,0 mol) 2-Propylheptansäure (8) und 1,74 g Kaliumhydroxid (50 Gew.% in Wasser) wurden bei 90 °C und unter 20 mbar am Rotationsverdampfer unter Stickstoff entwässert. Danach wurde der Ansatz in einen Druckreaktor überführt, der Druck mit Stickstoff auf 1,5 bar eingestellt und 88,0 g (2,0 mol) Ethylenoxid (4) innerhalb 1 h bei 140 °C zudosiert mit einem Druck von maximal 7,2 bar. Nach 12 h Nachrühren ließ man den Ansatz auf 80 °C abkühlen, spülte unter Rühren mit Stickstoff und erhielt 441 g eines hellgelben, klaren Produkts (9). Die H-NMR Analyse bestätigte die Struktur.

### Beispiel 2 - Formulierung von Epoxyconazol

5,2 bzw. 10,4 g Epoxiconazol, 7,5 g Tensid 1, 7,5 g Tensid 2 und 12,5 g Benzylalkohol wurden eingewogen und mit Alkoxyalkanolat aus Beispiel 1 auf 100 ml Gesamtvolumen aufgefüllt. Der Ansatz wurde durch Rühren bei Raumtemperatur so lange vermischt, bis eine klare, homogene Lösung von Epoxiconazol erhalten wurde.

| Versuch | Menge Epoxiconazol | Alkoxyalkanolat aus Beispiel |
|---|---|---|
| A | 5,2 g | 1 A |
| B | 5,2 g | 1 B |
| C | 5,2 g | 1 C |
| D | 5,2 g | 1 D |
| E | 10,4 g | 1 A |
| F | 10,4 g | 1 B |
| G | 10,4 g | 1 C |
| H | 10,4 g | 1 D |

Eine Probe der Versuche A bis F wurde jeweils mit CIPAC Wasser D (enthaltend 342 ppm Ca/Mg Ionen) auf eine 1 Gew.%ige Emulsion verdünnt und sechs Stunden bei 20 °C stehen gelassen. Während dieser Zeit bildeten sich keine Kristalle von Expoxiconazol.

### Beispiel 3 - Formulierung von Pyraclostrobin

25,2 g Pyraclostrobin, 5,0 g Tensid 3 und 5,0 g Tensid 4 wurden eingewogen und mit Alkoxyalkanoat aus Beispiel 1 auf 100 ml Gesamtvolumen aufgefüllt. Der Ansatz wurde durch Rühren bei Raumtemperatur so lange vermischt, bis eine klare, homogene Lösung von Pyraclostrobin erhalten wurde.

| Versuch | Alkoxyalkanoat aus Beispiel |
|---|---|
| A | 1A |
| B | 1B |
| C | 1C |
| D | 1D |

Eine Probe der Versuche A bis C wurde jeweils mit CIPAC Wasser D (enthaltend 342 ppm Ca/Mg Ionen) auf eine 1 Gew.%ige Emulsion verdünnt und sechs Stunden bei 20 °C stehen gelassen. Während dieser Zeit bildeten sich keine Kristalle von Pyraclostrobin.

### Beispiel 4 - Formulierung von Metconazol

15,2 g Metconazol, 5,0 g Tensid 3 und 5,0 g Tensid 4 wurden eingewogen und mit Alkoxyalkanoat aus Beispiel 1 auf 100 ml Gesamtvolumen aufgefüllt. Der Ansatz wurde durch Rühren bei Raumtemperatur so lange vermischt, bis eine klare, homogene Lösung von Metconazol erhalten wurde.

| Versuch | Alkoxyalkanoat aus Beispiel |
|---|---|
| A | 1A |
| B | 1B |
| C | 1C |
| D | 1D |

Eine Probe der Versuche A bis C wurde jeweils mit CIPAC Wasser D (enthaltend 342 ppm Ca/Mg Ionen) auf eine 1 Gew.%ige Emulsion verdünnt und sechs Stunden bei 20 °C stehen gelassen. Während dieser Zeit bildeten sich keine Kristalle von Metconazol.

## Patentansprüche

1. Flüssige, agrochemische Zusammensetzung umfassend Pestizid, Formulierungshilfsmittel und Alkoxyalkanoat, **dadurch gekennzeichnet, dass** das Alkoxyalkanoat der Formel I
R¹-C(O)-(O-R²)ₙ-OH I
wobei R¹ für ein verzweigtes C₇-C₁₁-Alkyl,
R² für ein C₂-C₄-Alkylen, und
n für 1 bis 3 steht,
entspricht, wobei das Pestizid bei 20 °C zu mindestens 10 g/l löslich im Alkoxyalkanoat ist.

2. Zusammensetzung gemäß Anspruch 1, **dadurch gekennzeichnet, dass** mindestens 20 Gew.% Alkoxyalkoanoat der Formel I bezogen auf die Zusammensetzung enthalten sind.

3. Zusammensetzung gemäß Anspruch 1 oder 2, **dadurch gekennzeichnet, dass** n für 1 bis 2 steht.

4. Zusammensetzung gemäß den Ansprüchen 1 bis 3, **dadurch gekennzeichnet, dass** n für 1 steht.

5. Zusammensetzung gemäß einem der Ansprüche 1 bis 4, **dadurch gekennzeichnet, dass** R¹ für ein verzweigtes C₈ bis C₉ Alkyl steht.

6. Zusammensetzung gemäß einem der Ansprüche 1 bis 5, **dadurch gekennzeichnet, dass** das Pestizid in gelöster Form vorliegt.

7. Verfahren zur Herstellung einer Zusammensetzung gemäß den Ansprüchen 1 bis 6, **dadurch gekennzeichnet, dass** man ein Pestizid und ein Alkoxyalkanoat der Formel I mischt.

8. Alkoxyalkanoat der Formel V
R³-C(O)-(O-R⁴)ₙ-OH V
wobei R³ für eine Struktur der Formel G, H oder J 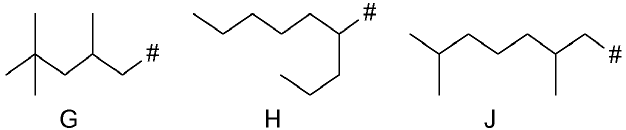 steht, wobei # die Bindung zur Carbonylgruppe von Formel V kennzeichnet,
R⁴ für ein C₂-C₄-Alkylen, und
n für 1 bis 3 steht.

9. Alkoxyalkanoat V gemäß Anspruch 8, **dadurch gekennzeichnet, dass** n für 1 bis 2 steht.

10. Alkoxyalkanoat gemäß Anspruch 8, **dadurch gekennzeichnet, dass** R³ für eine Struktur der Formel G oder H steht.

11. Verwendung der Alkoxyalkanoate gemäß Anspruch 8 bis 10 in agrochemischen Formulierungen.

12. Verwendung der Zusammensetzung gemäß einem der Ansprüche 1 bis 6 zur Bekämpfung von phytopathogenen Pilzen und/oder unerwünschtem Pflanzenwuchs und/oder unerwünschtem Insekten- oder Milbenbefall und/oder zur Regulation des Wachstums von Pflanzen, wobei man die Zusammensetzung auf die jeweiligen Schädlinge, deren Lebensraum oder die vor dem jeweiligen Schädling zu schützenden Pflanzen, den Boden und/oder auf unerwünschte Pflanzen und/oder die Nutzpflanzen und/oder deren Lebensraum einwirken lässt.

13. Verwendung der Zusammensetzung gemäß einem der Ansprüche 1 bis 6 zur Bekämpfung von unerwünschtem Insekten- oder Milbenbefall auf Pflanzen und/oder zur Bekämpfung von phytopathogenen Pilzen und/oder zur Bekämpfung unerwünschten Pflanzenwuchs, wobei man Saatgüter von Nutzpflanzen mit der Zusammensetzung behandelt.

14. Saatgut, gebeizt mit der Zusammensetzung gemäß einem der Ansprüche 1 bis 6.

## Claims

1. A liquid agrochemical composition comprising pesticide, formulation auxiliary and alkoxyalkanoate, wherein the alkoxyalkanoate has the formula I
R¹-C(O)-(O-R²)ₙ-OH I
where R¹ is a branched C₇-C₁₁-alkyl,
R² is a C₂-C₄-alkylene, and
n is 1 to 3,
where the pesticide is soluble in the alkoxyalkanoate to at least 10 g/l at 20°C.

2. The composition according to claim 1, wherein at least 20% by weight of alkoxyalkanoate of the formula I based on the composition is present.

3. The composition according to claim 1 or 2, wherein n is 1 to 2.

4. The composition according to any of claims 1 to 3, wherein n is 1.

5. The composition according to any of claims 1 to 4, wherein R¹ is a branched C₈ to C₉-alkyl.

6. The composition according to any of claims 1 to 5, wherein the pesticide is present in dissolved form.

7. A process for the preparation of a composition according to claims 1 to 6, wherein a pesticide and an alkoxyalkanoate of the formula I are mixed.

8. An alkoxyalkanoate of the formula V
R³-C(O)-(O-R⁴)ₙ-OH V
where R³ is a structure of the formula G, H or J
R⁴ is a C₂-C₄-alkylene, and
n is 1 to 3.

9. The alkoxyalkanoate V according to claim 8, wherein n is 1 to 2. 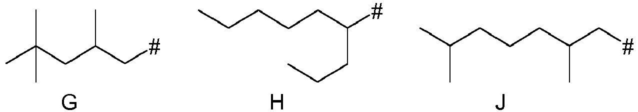 where # denotes the bond to the carbonyl group of formula V,

10. The alkoxyalkanoate according to claim 8, wherein R³ is a structure of the formula G or H.

11. The use of the alkoxyalkanoates according to claims 8 to 10 in agrochemical formulations.

12. The use of the composition according to any of claims 1 to 6 for controlling phytopathogenic fungi and/or undesired vegetation and/or undesired attack by insects or mites and/or for regulating the growth of plants, where the composition is allowed to act on the respective pests, their environment or the plants to be protected from the respective pests, on the soil and/or to undesired plants and/or the useful plants and/or their environment.

13. The use of the composition according to any of claims 1 to 6 for controlling undesired attack by insects or mites on plants and/or for controlling phytopathogenic fungi and/or for controlling undesired vegetation, where seeds of useful plants are treated with the composition.

14. Seed, dressed with the composition according to any of claims 1 to 6.

## Revendications

1. Composition liquide, agrochimique, comprenant un pesticide, des adjuvants de formulation et un alcoxyalcanoate, **caractérisée en ce que** l'alcoxyalcanoate correspond à la formule I
R¹-C(O)-(O-R²)ₙ-OH I
dans laquelle
R¹ représente C₇-C₁₁-alkyle ramifié,
R² représente C₂-C₄-alkylène et
n vaut 1 à 3,
le pesticide étant soluble dans l'alcoxyalcanoate à 20°C à raison d'au moins 10 g/l.

2. Composition selon la revendication 1, **caractérisée en ce qu'**elle contient au moins 20% en poids d'alcoxyalcanoate de formule I par rapport à la composition.

3. Composition selon la revendication 1 ou 2, **caractérisée en ce que** n vaut 1 à 2.

4. Composition selon les revendications 1 à 3, **caractérisée en ce que** n vaut 1.

5. Composition selon l'une quelconque des revendications 1 à 4, **caractérisée en ce que** R¹ représente C₈-C₉-alkyle ramifié.

6. Composition selon l'une quelconque des revendications 1 à 5, **caractérisée en ce que** le pesticide se trouve sous forme dissoute.

7. Procédé pour la préparation d'une composition selon les revendications 1 à 6, **caractérisé en ce qu'**on mélange un pesticide et un alcoxyalcanoate de formule I.

8. Alcoxyalcanoate de formule V
R³-C(O)-(O-R⁴)ₙ-OH V
dans laquelle R³ représente une structure de formule G, H ou J 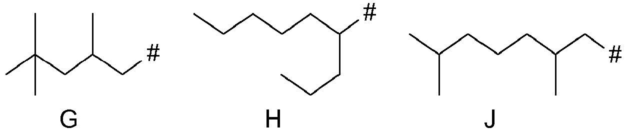
dans lesquelles # désigne la liaison au groupe carbonyle de formule V,
R⁴ représente C₂-C₄-alkylène et
n vaut 1 à 3.

9. Alcoxyalcanoate V selon la revendication 8, **caractérisé en ce que** n vaut 1 à 2.

10. Alcoxyalcanoate selon la revendication 8, **caractérisé en ce que** R³ représente une structure de formule G ou H.

11. Utilisation des alcoxyalcanoates selon la revendication 8 à 10 dans des formulations agrochimiques.

12. Utilisation de la composition selon l'une quelconque des revendications 1 à 6 pour lutter contre des champignons phytopathogènes et/ou une croissance non souhaitée de plantes et/ou une attaque non souhaitée par des insectes ou des acariens et/ou pour la régulation de la croissance de plantes, où on laisse agir la composition sur les différents organismes nuisibles, leur espace de vie ou les plantes, le sol à protéger contre les différents organismes nuisibles et/ou sur les plantes non souhaitées et/ou les plantes utiles et/ou leur espace de vie.

13. Utilisation de la composition selon l'une quelconque des revendications 1 à 6 pour lutter contre une attaque non souhaitée par des insectes ou des acariens sur des plantes et/ou pour lutter contre des champignons phytopathogènes et/ou pour lutter contre une croissance non souhaitée de plantes, les semences de plantes utiles étant traitées par la composition.

14. Semences, traitées par la composition selon l'une quelconque des revendications 1 à 6.
